# EUROPEAN PATENT APPLICATION

(11) **EP 1 637 148 A1**
(43) Date of publication of application: **22.03.2006**
(21) Application number: 03730214.8
(22) Date of filing: 05.06.2003
(51) Int. Cl.: A61K 36/00, A61P 15/10, A61P 43/00

(54) **NOVEL THERAPEUTIC USE OF POLYPODIUM EXTRACTS**

(30) Priority: 06.06.2002 ES 200201345
(71) Applicant: Especialidades Farmaceuticas Centrum, S.A., 03006 Alicante (ES)
(72) Inventor: QUINTANILLA ALMAGRO, Eliseo, 03006 ALICANTE (ES)
(86) International application number: PCT/ES2003/000272
(87) International publication number: WO 2003/103695

(57) **Abstract**

New therapeutic use of Polypodium extract. A new use of Polypodacea genus extracts is described for the treatment of fibrotic pathologies, such as, scleroderma, pulmonary fibrosis, atherosclerosis, bone marrow fibrosis, liver fibrosis, pancreatic fibrosis, kidney fibrosis, cardiac fibrosis, Dupuytrem's disease and, specially, Peyronie's disease; said fibrotic pathologies are produced by an extracellular matriz (ECM) overexpresion, and by a Transforming Growth Factor (TFG-β) overexpression. It is disclosed extracellular matriz inhibition in vitro; and penis tunica albuginea reduction and plaque redution in patients who suffer Peyronie's diseaseas after Polypodium extracts administration. In a preferred mode, Polypodium extracts are obtained from Polypodium leucotomos rhizomes and extraction with a polar solvent.

## Description

### Technical field of the invention

The present invention describes a new therapeutic of the Polypodium extracts, concretely, for the treatment of fibrotic conditions.

### Background of the invention

Tissular fibrosis are chronic pathologies which are characterized by an over expression of extra cellular matrix (ECM), mainly collagen and fibronectin, and fibroblast proliferation.

Recently, it has been discovered that Transforming Growth Factor- beta (TGF-β) is involved in all fibrotic conditions, and (TGF-β) induces ECM protein expression, independently of the disease. For instance, scleroderma, liver fibrosis, bone marrow fibrosis, cardiac fibrosis, pulmonary fibrosis, atherosclerosis, pancreatic fibrosis, kidney fibrosis, Dupuytren's disease or La Peyronie's disease, may be cited as fibrotic disorders.

With regard to Peyronie's disease, its history, its ethyology and its epidemiology are unknown. Genetic factors, traumatic factors, arterial hypertension and/or idiopathic factors may be considered as etiological factors in Peyronie's disease.

Clinically , Peyronie's disease patients present a formation of fibrous in the tunica albuginea of penis producing painful scars in the earlier stages of the disease. The plaques during erection produce an incurvature in the penis which in advanced stages make difficult or prevent sexual activity. In certain occasions, the clinical symtoms are accompanied by loss of rigitity in erection.

Like other fibrotic diseases, it has been reported that (TFG-β) is involved in Peyronie's diseases, and this protein is increased in Peyronie's patients. Further, habitually Peyronie's disease is associated with other fibrosis, mainly Dupuytren's disease or Paget's disease.

The searching of new treatments for fibrotic diseases focused to products which may inhibit Transforming Growth Factor (TFG-β), and this protein would reduce ECM protein synthesis. However,there is no clear correlation between TFG- β reduction in vitro studies and animals models, with the results obtained in vivo with respect to the clinical improvement or collagen reduction in the plaque.

El-Sakka et al. (J. Uro. 1999 Jun;161(16):1980-3) teach that colchicin reduces TFG-β expression in rats that developed Peyronie's disease by injection of TGF- β in their penis.

However, Reinhart et al. (Dtsch Z Verdau Stoffwechselkr 1986;46(5):257-75) describe that treatment with colchicin in liver fibrosis gives with equal frequency an improvement, worsening or no change in fibrotic grades.

With regard to Peyronie's disease; (Kadioglu et al. Int. J. Impot. Res. 2000 Jun; 12(3):169-75) teach that 60 patients suffering Peyronie's disease were treated for 10 months with colchicin, penis incurvature was improved in 30%, was worse in 22% and no changes was reported in 48%.

Nowadays, Tamoxifen is one of therapies in Peyroine's disease according to Tamoxifem activity for down-regulating TGF-β in vitro (Ralph et al. *The treatment of Peyronie's disease with Tamoxifen.* Br J Urol. 1992,70: 684-651). Nevertheless, the clinical effectiveness of tamoxifen is low. Thus, Teloken C. et al *(The* Tamoxifen *versus placebo in the treatment* of *Peyroniés diseases,* J Urol. 1999 Dec;162(6):2003-5),disclose that the results were similar in a comparative clinical study between Tamoxifen versus placebo.

Other methods to reduce the plaque include the injection via intracavernous of diverse pharmacological agents, for instance:interferón α-2b, verapamil or corticoids. This route of administration is painful for the patient and the administration of the drug must be carried out by a qualified person. The administration of these agents can produce adverse effect: inferferon gamma produces fever, malaise and mialgia; corticoids intraplaque produce gangrene; tamoxifen administred orally produces diminution of sexual desire and diminution of the eyaculate.

Polypodium extract have been described in the prior art having different pharmacological activities.EP-0503.208, ES-2.088.770, EP 1172111 and prior art of EP 1172111 describe different pharmacological activities of Polypodium extracts. Said pharmacological activities may be summarized as: immunological activity and regulation of different cytokine populations; regulation of adhesion molecules; activity neurodegenerative disease;anti-inflamatory activity and collagenpoietic activity.

So, the closet prior art describes collagenpoiectic activities of Polypodium extracts and their uses in pathologies which are characterized by an collagen deficit.

EP-503.208 discloses the activity of Polypodium extracts to stimulate fibroblasts and to synthesize precollagen.

Also,FR 2.479.690 discloses the use of Polypodaceas extracts in the treatment of the diseases of osteomotor system that attend a collagen deficit. Thus, this document suggests that Polypodium extracts increase collagen synthesis and that formed collagen is more resitant.

### Summary of the invention

The problem solved in the present invention is to achieve an effective pharmacological treatment for fibrotic diseases, such as scleroderma, pulmonary fibrosis, kidney fibrosis, atherosclerosis, pancreatic fibrosis, cardiac fibrosis, liver fibrosis, bone marrow fibrosis, Dupuytren's disease and, specially, Peyronie's disease, wherein said pathologies are characterized by an extracelullar matrix (ECM) overexpression.

The solution found by the inventors is the use of Polypodium extracts, as is cited in claim 1.

The inventors have found that Polypodium extracts inhibit in vitro in a dependent-doses manner collagen and fibronectin synthesis in human being. This capacity also has been shown in vivo; the Polypodium extracts have reduced collagen plaque in patients who suffering Peyronie's disease, a fibrotic disease which is characterized by fibrosis in penis.

Another problem solved by the Polypodium extracts is that said extracts reduce Transforming Growth Factor-beta (TGF-β) expression. This protein is involved in all fibrotic conditions, as is cited in claim 2. Further, the TGF- β reduction produced by Polypodium extracts in vivo is more significant than TGF- β reduction produced by tamoxifen, the reference drug for regulating TGF- β,

As is known by the skilled person, administration of Polypodium extracts produces no adverse effects, therefore, adverse effects produced by anti-cytokine therapies are avoided.

In a preferred embodiment, other problem solved by Polypodium administration is to improve clinical parameters related to Peyronie's disease, that is; after oral administration of Polypodium extracts, fibrotic plaque in penis tunica albugenia, pain during sexual act, penis incurvature are reduced, and an psychological improvement in patients is also shown, according to claim 3. The obtained clinical results in Peyronie's patients who were treated with Polypodium extracts were better than the obtained results with other drugs.

Oral administration, according to claim 9, solves the problems related by injecting into Peyronie's plaque administration: Said injection must be performed by a skill man, and this administration dosage is painful.

In a particular embodiment, Polypodium extract are obtainable by extraction with a polar solvent having a dielectric constant higher than 20. Concretely, Polypodium extracts are obtainable by extraction with water, and more concretely, extracts are obtainable by water extraction from Polypodium leucotomos, as is cited in claim 4,5,6 and 7.

In order to describe the extracts parametrically, claim 8 relates the chemical compounds present in the extracts.

### Detailed description of the invention

Polypodium extracts are obtainable by extraction with a polar solvent, meaning " polar solvent" a solvent having a dielectric constant (E) higher than 20. Following table shows some dielectric constants according to Handbook of Chemistry and Physics, 54^{a} Edition.1973

| Solvent | Dielectric constant (∈) |
|---|---|
| Water | 80 |
| Methanol | 32 |
| Ethanol | 24 |
| Ethyleneglycol | 37 |

In a preferred embodiment, Polypodium extracts are obtained by water extraction and solvent evaporation.

Alternatively, it is possible to use other known operations for the expert in the matter and described in the state of the art for the purification of the Polypodium extracts , or to be improved the yield of the extraction. Therefore, these operations would not contribute any additional advantage to the extract. These operations may be: optimization of the drying conditions of the plant; solvent combinations ; defatting of the vegetal material; addition of preservatives; addition of antimicrobial agents; addition of carriers to facilitate the conservation and to improve the manipulation; extraction with supercritical fluid state solvents ; optimization of the time of harvesting of the vegetal material; purification by means of chromatography; purification by means of adsorption with activated charcoal or others.

The described vegetal material in the invention was collected in the region of Montes Mataque in Guatemala, with the consequent authorization of the authorities of the country.

The taxonomical classification of the vegetal species depends on the author, therefore, different nomenclatures are used depending on the classification

The taxonomical classification of the used vegetal material is :
Scientific name: Phlebodium aureum (Linnaeus)
Subgenus: Phlebodium
Genus: Polypodium
Family: Polypodiaceae

Further, other accepted synonymies exist, such as, Polypodium aureum (Linnaeus) or Polypodium leucotomos (Poiret) and other varieties: Polypodium aerolatum, Phlebodium aerolatum, Polypodium aureum var. Aerolatum or Pleopeltis aurea.

Therefore, "Polypodium" concerns to the ferns included in Polypodium genus.

In a preferred embodiment, rhizomes from Polypodium leucotomos (Poiret) are dried and extrated with water wherein the ratio rhizome/water is 1:10. In order to reduce microbiological content, the extract is filtrated throught membrane. The solvent is removed at low pressure to obtain an extract having a water content about 25%. In order to avoid microbiological contamination and to facilitate the manipulation, carriers are added into extract ( starch, lactose, magnesium stearate). The stabilized extract is capsulated in unitary doses having 120 mg of dried water extract.

The chromatographyc analysis of the extracts ( Columm coated with octadecylsilane, 5 micrometers; Mobile phase (3% Phosphoric acid: Water: Acetonitrile/ 25:72:3); detection UV 210 nm) shows the presence of malic acid, citric acid, fumaric acid, quinic acid and lactic acid. These acids are used as markers of the extract for analytical purposes.

Human skin fibroblast cultures are suitable model for studying of drug in fibrotic diseases, that is, the patologies are characterized by an extracellular matrix (ECM) overexpression.

Polypodium extracts have inhibited collagen synthesis in a dose dependent manner at concentrations of 1,10, 50, 100, 500 µg/ml in presence of tritiated proline (³H-proline) at 37°C for 24 hours. Polypodium extracts at concentration of 50 µg/ml inhibited collagen synthesis to 40% of control values.

Polypodium extracts also have inhibited fibronectin synthesis ,in vitro, both leaf extracts and rhizome extract inhibited synthesis fibronectine.After fibroblast incubation in presence of Polypodium extracts and immunoassay, Polypodium extracts inhibited in a dose dependent manner (20 -1000 µg/ml) up to 40% of controls fibronectin synthesis.

In addtion to the inhibiting activy of ECM synthesis, in vitro, Polypodium extracts have also shown said actity in vivo. After administration of Polypodium extracts to Peyronie patients in vivo, the collagen penis plaque was reduced, and TFG-β expression was reduced too.

A clinical trial on 34 Peyronie's patients for 6 months showed that clinical and psychological improvement were better in Polypodium group than tamoxifen group. The obtained results were statically significant.

The results were:

| | GRUP P Polypodium | | | GRUP T Tamoxifen | | |
|---|---|---|---|---|---|---|
| N° Patient | 19 | | | 15 | | |
| Parameters | Improv | Same | Worse | Improv | Same | Worse |
| Plaque | 5 (26%) | 14 (74%) | 0 | 4 (27%) | 8 (53%) | 3 (20%) |
| Pain | 13 (68%) | 6 (32%) | 0 | 10 (66%) | 5 (33%) | 0 |
| Penis incurvation | 13 (68%) | 5 (26%) | 1 (5%) | 7 (46%) | 5 (33%) | 3 (20%) |
| Coital difficulty | 8 (42%) | 6 (33%) | 5 (25%) | 6 (40%) | 3 (20%) | 6 (40%) |

The results after treatment with Polypodium extract were better than the results disclosed by Kadioglu et al, (Int. J. Impot. Res. 2000 Jun; 12(3):169-75). These autors teach that 60 Peyronie's patients treated for 10 months with colchicin, penis incurvature improved in 30%, remained unchanged in 48% and deteriorated in 22 %.

The patients of Polypodium group were treated with 720 mg of Polypodium extract, orally, at 8-hourly intervals.

The patients of Tamoxifen group were treated with 20 mg of Tamoxifen, orally, at 24-hourly intervals.

The age was similar in both groups.

The plaque was assessed by physical examination and penis ecography.

Penis incurvation was assessed by photograph.

A 22% post treatment decrease in TGF-β production was found in Polypodium group when compared with initial values, against 5% in Tamoxifen group.

Also, a 17% post treatment decrease in IL-10 production in Polypodium group when compared with initial values, against 79% in Tamoxifen group.

After treatment, 67% of Polypodium group was satisfied against 45 % of Tamoxifen group.

### Brief explanation of figures

Figure 1. Collagen synthesis inhibition produced by Polypodium extracts. Every value represents the mean of 4 parallel experiments. The standard deviations are indicated.

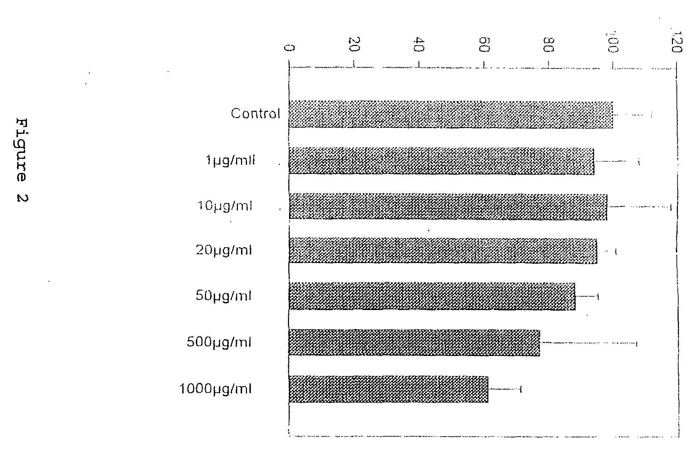

Figure 2.Fibronectin synthesis inhibition produced by Polypodium extracts. Every value represents the mean of 3 parallel experiments. The standard deviations are indicated.
Figure 3. Fibronectin synthesis inhibition produced by Polypodium extracts. Every value represents the mean of 3 parallel experiments. The standard deviations are indicated.

### Example 1. Effects of Polypodium extract on extracellular matrix.

Polypodium extracts. Polypodium extracts were obtained by water extraction for 48 hours, filtration through membrane, solvent removing at low pressure, redissolution in physiological serum and dry-freezing. The extracts were dissolved in PBS and they were used after reconstitution.

Cell cultures. Fibroblasts were obtained from surgical material. The skin pieces were pre-incubated for 2 hours at 4°C in RPMI 1640 medium and 2% (double amount) of penicillin/streptomycin solution. The fatty tissues were removed, the skin were cut in small pieces and fixed to micro-wells, which had been dampened with fetal calf serum (FCS). The skin cell were cultivated in a 5% CO₂ - atmosphere in RPMI 1964 medium with 10% FCS and 1% penicillin/streptomycin solution. The medium was renewed twice a week. The fibroblasts were tripsinizated (Tripsin/EDTA: 0.05%/0.02%) and were subcultured for the experiments. Cells obtained from 4^{th} to 14^{th} passage were only used.

Collagen synthesis. Human skin fibroblasts were harvested in tissue culture micro plates. Each microwell was seeded with 10.000 cells in 100 µl of RPMI medium with 10 % FCS and 50 µg/ml L-ascorbic acid. After incubation for 24 hours in 5 % carbon dioxide atmosphere, the medium was renewed by 100 ml of fresh medium containing the different concentrations of Polypodium extracts (1- 1000 µg/ml) and 1µCi ³H-proline. A later incubation for 24 hours, the collagen was extracted by the addition of 100 µl of cold 1M acetic acid with 1% pepsin; the medium was cultured overnight at 4°C. The following steps were carried out at 4°C. The content of the microwells were transferred to 3-ml polypropylene tubes. 800 ml of 0.5M acetic acid containing a collagen soluble salt of rat skin as diluents was added. The tubes were centrifuged at 4000g for 20 minutes. The supernatant were transferred to clean tubes and the precipitates were discarded. The collagen was precipitated by addition of 250 µl of 25 %sodium chloride solution in 0.5M acetic acid. The tubes were centrifuged at 4000 g for 2 hours. The precipitates were redissolved in 0.05 M Tris-HCl solution containing 0.15 moles of sodium chloride, pH 7.5. The collagen was precipitated by the addition of 2 ml of 4.5 M. Two hours later, the tubes were centrifuged at 4000 g for 30 minutes. The supernatant was discarded and precipitated collagen was washed with 2 ml of 2% ethanol.
The resulting solution was centrifuged at 4000g for 30 minutes. Finally, each precipitated was dissolved in 250 µl of 0.5 M acetic acid. The solution was transferred to scintillation vials containing 5-10 ml of scintillation solution. The radioactivy was measured using an external standard in a liquid scintallion counter, according to method described by Webster and Harvey, Analytical Biochemistry 96,220-224,1979.

Fibrotecnin synthesis. The fibroblasts were cultured in micro well plates, the initial density was 20.000 cells/ml, and the medium was RPMI without FCS. The cells were cultured for 24 hours at 37 °C in 5% dioxide carbon atmosphere. The medium was renewed. Different concentrations of Polypodium extract were added to the cultures (1 -1000 µg/ml). Later, the cells were washed tree times with PBS containing 1% bovine serum albunin (BSA) and y polysorbarte 20 (Tween 20). The cells were fixed with water/acetone (v/v:1/1) for 30 minutes, and washed three times as is cited above. The cells were incubated with Monoclonal Anti-Fibronectin antibody produced in mouse(Sigma F-7384). After washing, the cells were incubated with Anti-Mouse IgG (Fab specific)-Alkaline Phosphatase antibody produced in goat for 1 hour at 37°C. Cells were washed three times again, and they were incubated with a solution of p-nitrophenyl phosphate, having a concentration of 1 mg/ml, for 15 minutes in darkness.

Then, microplates were centrifuged at 200g for 5 minutes and 100 µl of supernatant were transferred to a new micro plate. An ELISA reader measured absorbance at 405 nm.

### Results

### 1. Effects of Polypodium extracts on collagen synthesis inhibition.

The results obtained were:

| Concentration % Control | incorporation 100% | % inhibition 0% |
|---|---|---|
| 1 µg/ml | 116% | -16% |
| 10 µg/ml | 84% | 16% |
| 50 µg/ml | 58% | 42% |
| 100 µg/ml | 60% | 40% |
| 500 µg/ml | 53% | 47% |
| 10³ µg/ml | 64% | 36% |

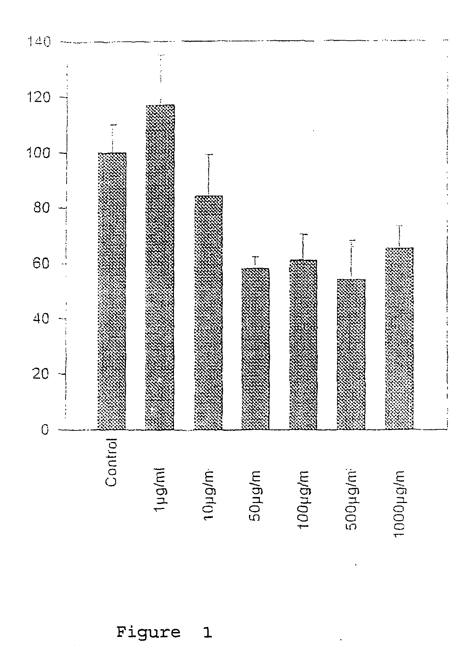

Figure 1 shows these results.

### 2. Effects of Polypodium extracts on fibronectin inhibition synthesis.

The obtained results on 3 pararell experiments were:

| Concentration Control | % of incorporation 100% | % of inhibition 0% |
|---|---|---|
| 1 µg/ml | 95% | 5% |
| 10 µg/ml | 98% | 2% |
| 20 µg/ml | 95% | 5% |
| 50 µg/ml | 87% | 13% |
| 500 µg/ml | 76% | 24% |
| 10³ µg/ml | 60% | 40% |

### 3. Effects of Polypodium extract from leaves on fibronectin synthesis inhibition.

The obtained results on 3 pararell experiments were:

| Concentration Control | % of incorporation 100% | % of inhibition 0% |
|---|---|---|
| 1 µg/ml | 105% | -5% |
| 10 µg/ml | 86% | 14% |
| 20 µg/ml | 85% | 15% |
| 50 µg/ml | 82% | 18% |
| 500 µg/ml | 55% | 45% |
| 10³ µg/ml | 40% | 60% |

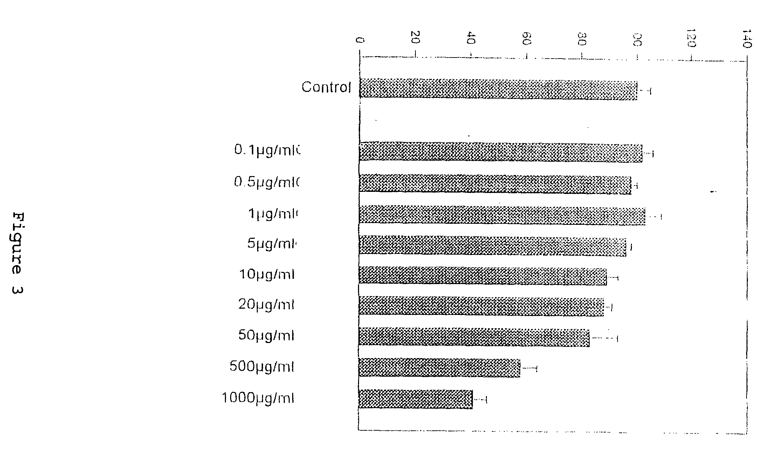

Figure 3 shows these results.

## Claims

1. Use of the Polypodium extracts for the manufacture of a pharmaceutical composition for the treatment of fibrotic diseases, such as, scleroderma, pulmonary fibrosis, atherosclerosis, bone marrow fibrosis, kidney fibrosis, cardiac fibrosis, Dupuytren's diseases, Peyronie's disease.

2. Use of Polypodium extract according to claim 1, **characterized by** said extracts reduce Transforming Growth Factor-beta (TGF-β) expression.

3. Use of the Polypodium extracts according to any of claim 1-2, **characterized by** said extracts, at least, reduce penis incurvature, reduce penis plaques, reduce coitus pain or produce an psychological improvement, in patients who suffer Peyronie's disease.

4. Use of the Polypodium extracts according to any of claims 1-3 wherein the pharmaceutical composition comprises an Polypodium extract which is obtainable by extraction using an solvent having a dielectric constant more than 20.

5. Use of Polypodium extracts according to claim 4 wherein the pharmaceutical composition comprises an extract, which is obtainable by extraction with means for dissolving water-soluble compounds.

6. Use according to any of preceding claims wherein the extracts are obtained from rhizomes.

7. Use according to claim 6 wherein extract are obtained from Polypodium leucotomos (Poiret).

8. Use according any of preceding claims **characterized by** the extracts comprises at least lactic acid, fumaric acid, quinic acid, citric acid or malic acid.

9. Use according to any of preceding claims **characterized in that** the pharmaceutical composition is orally admistrated.
